# EUROPEAN PATENT SPECIFICATION

(11) **EP 3 681 334 B1**
(45) Date of publication and mention of the grant of the patent: **14.04.2021**
(21) Application number: 18759140.9
(22) Date of filing: 31.08.2018
(51) Int. Cl.: A45D 2/36, A45D 20/08

(54) **HAIR STYLING DEVICE**
HAARSTYLINGVORRICHTUNG
DISPOSITIF DE COIFFURE

(30) Priority: 10.09.2017 EP 17190265; 25.01.2018 EP 18153518
(43) Date of publication of application: 22.07.2020
(73) Proprietor: Koninklijke Philips N.V., 5656 AG Eindhoven (NL)
(72) Inventor: SPOORENDONK, Wouter Hendrik Cornelis, 5656 AE Eindhoven (NL); ZJIROECHA, Nikolaj Vasiljevitsj, 5656 AE Eindhoven (NL); COSTECALDE, Laurentine, 5656 AE Eindhoven (NL)
(74) Representative: Philips Intellectual Property & Standards
(86) International application number: PCT/EP2018/073508
(87) International publication number: WO 2019/048357

(56) References cited:
- WO-A1-2010/067323
- WO-A1-2010/067323
- WO-A1-2014/108775
- WO-A1-2014/108775
- WO-A1-2017/080957
- WO-A1-2017/080957
- US-A1- 2015 173 480
- US-A1- 2015 173 480
- US-B1- 6 423 942
- US-B1- 6 423 942

## Description

### FIELD OF THE INVENTION

The invention relates to a hair styling device for e.g. hair crimping, curling, perming and straightening.

### BACKGROUND OF THE INVENTION

WO 2014/108775 discloses a system for straightening hair using hair straightening formulation solution vapors, comprising: a hair straightening iron connected to an electricity source, said hair straightening iron comprising a pair of pressing members each having a heating block on its inner surface, the pressing members joined by a hinge, the heating blocks comprising holes connected to vapor conducting tunnels accommodated inside each one of the pressing members; a vapor conducting pipe connected to the tail end of the iron, said vapor conducting pipe opens to the vapor conducting tunnels; and a vaporizer connected to said vapor conducting pipe on its other end, said vaporizer comprising a heating element connected to a source of electricity and a container configured to be heated by said heating element. In certain embodiments of the present invention the hair is heated by means of radiation of visible or invisible light from light sources. The straightening iron may comprise transparent windows for passing the light from light sources located behind the windows. As in most light sources, most of the electrical energy is converted to heat. Typically for LEDs the watt-to-watt efficiency, namely power of emitted light vs electrical power input, ranges from 40% for red light to 15% for green light. To operate the light source at high efficiency and prevent source damage heat needs to be removed. The process of heat dissipation from the light source can be viewed as accomplished in two steps: The first involves the transport of thermal energy away from the source to an external heat sink that absorbs the intense thermal energy. In the second stage the heat is dissipated into the surrounding environment. This is primary accomplished by either natural or forced air convection. Forced air convection typically involves an electric fan responsible for cooling the heat sink. The resulted heated air is further conveyed through air conducting channels and forced through ventilation openings located at the blades of the straightening iron. The resulting hot air can be further utilized for heating the hair.

WO2017080957A1, incorporated herein by reference, discloses a hair styling device comprising a heat source for heating hair up till a first temperature that is no more than 150 °C, and a radiation source for - in combination with heat from the heat source - selectively heating a hair cortex to a second temperature exceeding the first temperature and sufficiently high for hair styling. The heat source may be formed by e.g. hot plates which are directly or indirectly heated to a first temperature of e.g. 120 °C that is lower than a critical temperature for cuticle damage. The radiation source may be formed by e.g. a Continuous Wave, pulsed laser source, or an Intense Pulsed Light device with a low pass filter, for emitting a wavelength range between 400 and 600 nm, and preferably between 450 and 550 nm, which will selectively heat the cortex to a second temperature of about 170 °C that is sufficiently high for styling, for a duration of about a few milliseconds to a few seconds.

### SUMMARY OF THE INVENTION

It is, inter alia, an object of the invention to provide an efficient hair styling device. The invention is defined by the independent claim. Advantageous embodiments are defined in the dependent claims.

Embodiments of the invention provide a hair styling device comprising a heat source for heating hair by conduction via direct contact between a hair contact surface of the heat source and the hair, and an optical radiation source for - in combination with heat from the heat source - heating the hair to a temperature sufficiently high for hair styling by absorption of optical radiation by the hair, in which the heat source obtains heat from energy provided by the optical radiation source, and in a preferred embodiment, only from the optical radiation source. Advantageously, the heat source may include a heat bridge coupled to a heat sink of the optical radiation source, the heat bridge having the hair contact surface for heating the hair. The optical radiation source may advantageously be covered by a cover that is not fully transparent, whereby optical radiation energy is transformed into thermal energy, the heat source including the cover the cover having the hair contact surface for heating the hair. The cover may advantageously be largely transparent for wavelengths effective for hair styling, while the cover is largely not transparent for wavelengths less effective for hair styling. Advantageously, the optical radiation source may be covered by a cover that is heated by the heat source.

These and other aspects of the invention will be apparent from and elucidated with reference to the embodiments described hereinafter.

### BRIEF DESCRIPTION OF THE DRAWINGS

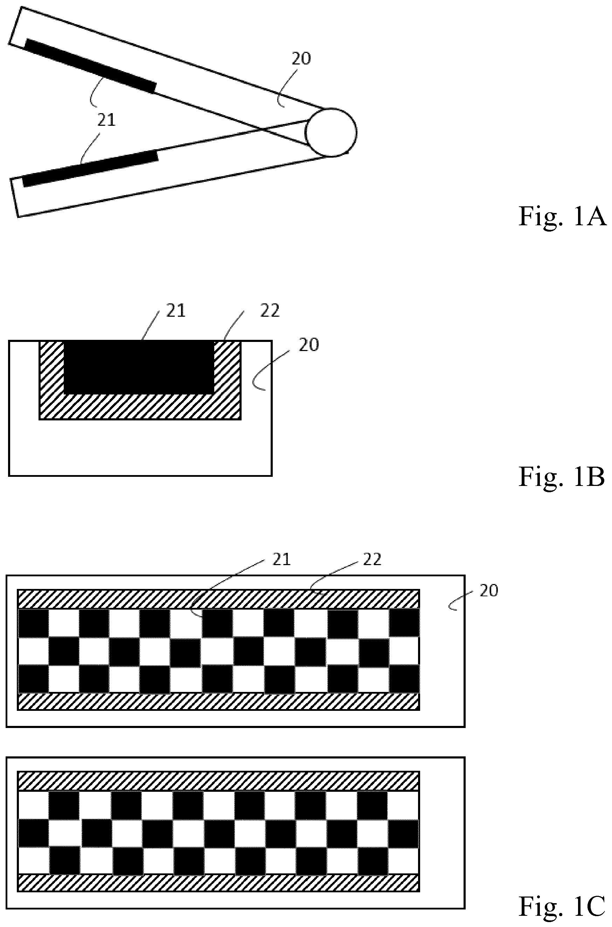

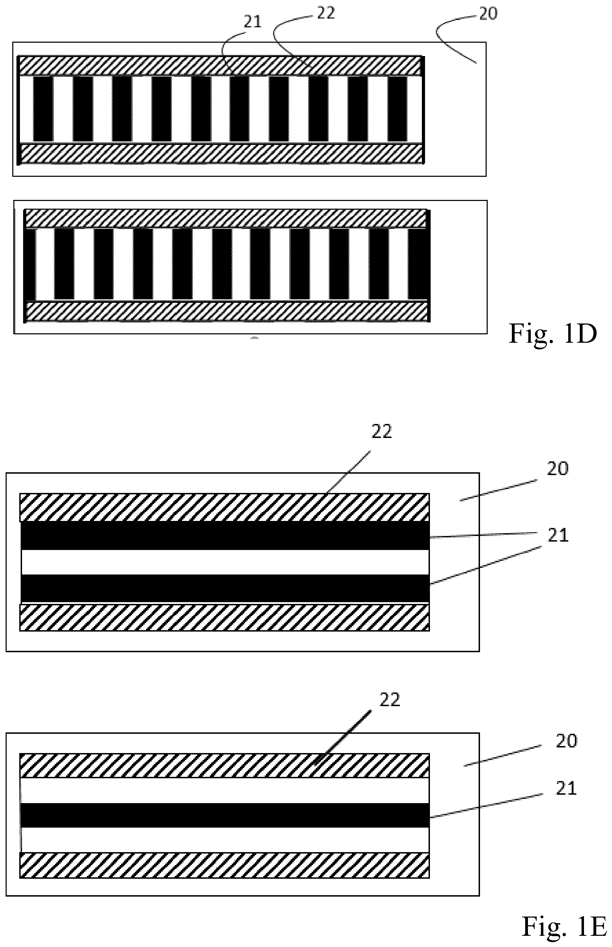

Figs. 1A - IE, 2A - 2B, 3 and 4 show various embodiments of the invention.

### DESCRIPTION OF EMBODIMENTS

An embodiment of the invention features a handheld hair styling device of the type disclosed in applicant's earlier application EP3216368 which comprises:
a pulse-driven light emitting diode (LED) or an array of LEDs configured to deliver optical energy to hair, wherein:
   an output wavelength is in the range 400 - 900 nm, with good results in the range 400 - 650 nm, and preferably in the range 450 - 550 nm,
   a pulse width is in the range 50 - 300 ms, preferably between 50 and 200 ms, such as in the range 100 - 200 ms, or between 50 and 100 ms,
a LED pulse driver circuit to drive the LED/s,
a control system to control the LED pulse driver, particularly controlling pulse electrical parameters including voltage, pulse duration, and pulse duty cycle,
a hair contacting interface configured to contact the hair and hold the hair in a pre-configured shape, e.g. planar, cylindrical, during pulsed light exposure provided by the LED, and
an optical shield configured to block stray light during light exposure of hair. The optical shield is configured to provide maximum recycling of light escaping from the hair lock, for instance by configuring the inner surface to be reflective and configured to have a parabolic shape.

A wavelength range between 400 and 900 nm, and preferably between 450 and 550 nm, appears to be the optimal wavelength range for selective heating of hair. However, high brightness high efficiency LEDs outputting light in the range between 800 nm to 1000 nm may result in a better penetration depth. Although at such higher wavelengths, the absorption is relatively lower than using lower wavelengths, styling by means of such LEDs emitting light in the range between 800 nm to 1000 nm could be more cost-effective than using high power near infrared LEDs.

The pulse width may be up to 1.5 s to achieve the required fluence with medium-power LEDs.

A thermal relaxation time constant of a hair volume to be heated at once appears to be around 200 ms.

In an experiment, a lock of brown hair was wound around a metal rod (diameter 15 mm) to a 132-unit array of 650 nm LEDs with energy fluence of 3 J/cm² with a pulse width of 100 ms. This resulted in a clear curling effect.

Light selectively heats up the hair, in particular the melanin of the hair, by absorption within a certain range of wavelengths (preferably between 400 and 900 nm and more preferably between 400 and 550 nm) and within a short period of time (preferably shorter than 300 ms). After the exposure of a light pulse, the hair temperature will increase depending on its volume, absorption rate and initial temperature.

At the beginning of the treatment, the hair is at room temperature (e.g. 20 °C). Next, after an intensive light pulse, the hair temperature increases. Temperature increases of 180 °C can be realized within a time frame of 0.05 - 1.5 s. However, within a time frame of about 0.1 s the light flash to achieve this temperature increase requires a lot of optical power. In most cases, the LED engine (of a consumer device) will not be powerful enough to do so. Moreover, if the LED is flashed twice or at a longer pulse duration (> 0.3 s), the same temperature increase can be realized with much lower power requirements and thus lower costs.

Light emitting diodes (LED) configured to deliver optical energy to hair, also produce heat. While LEDs are known to be very energy efficient, e.g. for blue light, using the most energy-efficient LEDs [2017], about 50% of the electrical input will become optical energy. However, while this is great compared to other light sources, it still means that the other 50% of the input energy becomes waste (heat)! In accordance with the principles of the present invention, this heat does not have to be wasted. While the thermal diffusion rate may be lower for conduction than for absorption, every form of heat applied towards the hairs is beneficial as it may be used to reduce the optical requirements.

So, as the LEDs not only produce optical energy but also thermal energy, which must be removed from the LEDs by means of heat sinks, this heat can be advantageously used in a useful way to pre-heat the hair before the hair is exposed to the light. As a result, if by doing so the hair is pre-heated to a temperature of e.g. 60 °C, the LEDs only need to heat up the hair from that 60 °C to a temperature sufficient for hair styling (i.e. above its glass transition temperature), instead of from room temperature (e.g. 20 °C) to that temperature sufficient for hair styling. So, the LEDs need to provide less optical energy to the hair, while the heat from the LEDs is efficiently re-used.

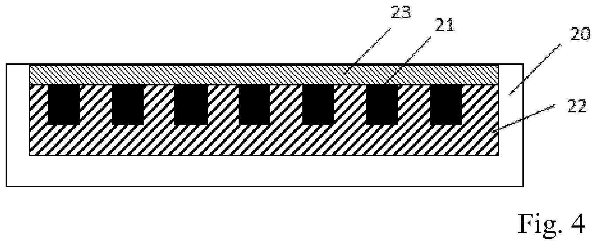

It is thus an aspect of the present invention to heat the hairs by absorption and conduction. This can be done in several ways. Firstly, heat the hairs by absorption (optical energy), and secondly feed the generated heat of the light source(s) towards the hairs by conduction. Or vice versa. In the end, this will lower the optical system requirements or at least compensates for any efficiency decrease during heating of the LEDs. In accordance with an embodiment of the invention, it is preferred to first heat the hairs by conduction using waste heat from the LEDs (e.g. from heat sinks), and only thereafter heat the hairs by absorption of light energy, as by doing so the contact surface does not have to be hot enough for getting a styling effect, and which temperature the hair also may become damaged. So, preheating the hair by means of conduction before the hair is exposed to light is much friendlier to the hair's health. Also, as it takes time to change the temperature of a heating plate, the system can much quicker adapt the final temperature if the heating plates only heat up till a temperature at which hair will not be damaged by the heat, while on top of that temperature, the heat level needed for hair styling is only achieved as a result of absorption of light energy which can be switched off almost instantaneously. For the same reasons, in a system that has conductive heating plates at both sides of the light source (e.g. as shown in Figs. 1B - 1E), in which the conducted heat is applied to the hair both before and after the light is applied to the hair, or which transforms light outside a bandwidth useful for hair styling into heat (e.g. as shown in Fig. 4), in which the conducted heat is applied simultaneously with the light to the hair, it is preferably still ensured that the contact surface for transferring heat by mean of conduction (in this case, e.g. a partially transparent cover of the LEDs) is at a temperature lower than a temperature needed for styling, while on top of that temperature, the heat level needed for hair styling is only achieved as a result of absorption of light energy.

In an embodiment, to photo-thermally heat hairs to a certain temperature an intense light source (LEDs) is flashed at a short distance from the hair (< 1 cm). This can be done with a short intense pulse (< 0.1 s) however this requires a lot of optical power (> 4 J/cm²). It is more efficient to heat the hair in multiple flashes or within a longer exposure time (> 0.3 s). For multiple flashes it is preferred to pulse again within the thermal relaxation time of the hair. This lowers the optical system requirements, and use can be made of the increased hair temperature as a new baseline. Directly after a light flash (< 5 s, more preferably < 1 s, most preferred < 0.3 s) the hair temperature (T₀) is increased. The temperature increase is only temporally: it decreases in time. Therefore, the shorter the interval between the flashes, the less time there is for the hair to cool down. As a result, the hair temperature is higher after two identical light flashes with a small interval (< 0.2 s) in-between compared to two individual light flashes with a longer interval (> 0.3 s) in-between.

It is thus a feature of an embodiment of the invention that the hairs are heated in two of multiple light flashes, in such a way that the light interval between subsequent flashes is preferably within the thermal relaxation time of the hair volume (< 0.3 s).

To heat the hairs within a longer pulse duration (> 0.3 s), the optical requirements can decrease, but in turn the diodes will heat up much more (around 100 °C) compared to small duty cycles (e.g. 0.1 s). The generated heat will, in the end, decrease the optical efficacy. Normally this heat is cooled down and/or dissipated in the environment (air) but now it is an aspect of this invention to recycle the heat loss of the light system. In one embodiment, this is done by creating a thermally isolated bridge between the heat sink of the LEDs and the treatment area. This way the generated heat of the light engine is fed into the hairs via conduction. This will lower the optical system requirements or at least compensates for any efficiency decrease during heating of the LEDs.

Embodiments of the invention thus provide that the heat sinks of the light sources are thermally connected to "hot plates" which are located next to the light emitting treatment areas to ensure that the heat loss of the light system is reused and guided to the hairs. In accordance with an embodiment of the invention, only the light sources produce heat.

Exemplary embodiments of the heat recycling of the LEDs, shown in Figs. 1A - 1E, feature a handheld hair styling device 20 in the form of a hair straightener, and comprise light exposure units 21 with arrays of light-emitting diodes (LEDs) inside. Fig. 1A shows a side-view of the hair straightener, while Fig. 1B shows a cross-section of one leg of the hair straightener. The heat sinks of the LEDs are thermally connected to a heat bridge 22 which surfaces next to the LED treatment area. In this embodiment, the hairs will be in contact with the heat bridge 22 before they are exposed to light. This way the hairs are heated both via the heat bridge 22 and the light exposure unit 21, and thus less optical power is needed, as the required styling temperatures (120 - 180 °C) are obtained by the combination of conduction heating by the heat bridge 22 and absorption of light from the light exposure unit 21. As the thermal diffusion rate of a hot plate is lower compared to photon absorption, the contact area of the heat bridge 22 is preferably as large as possible to allow as much hair temperature increase. In these embodiments, a heat source for heating hair by conduction via direct contact between a hair contact surface of the heat source and the hair includes the heat bridge 22. Figs. 1A - IE thus show an optical radiation source (formed by a light exposure unit 21 formed by LEDs) for - in combination with heat from the heat source (formed by heat bridge 22) - heating the hair to a temperature sufficiently high for hair styling by absorption of optical radiation by the hair.

To prevent LED light from shining in the eyes, the LEDs will only be powered if the device is closed, i.e. if the legs of the hair straightener of Fig. 1A are in a position in which the hair is clamped between the legs, which can be detected by means of a switch that is only pushed if the legs of this straightener touch one another.

Figs. 1C-1E shows advantageous layouts of the LEDs 21 in the upper and lower legs of the hair straightener 20, in a zone between parts of the heat bridge 22. This layout is based on the following considerations. In case no hair is clamped in-between the two legs of the hair straightener, or if the hairs do not cover the full treatment area, the LEDs in the upper and lower legs of the hair straightener will irradiate each other. The LEDs will absorb the light from the opposed member and will start to heat up, which will negatively affect the lifetime of the LEDs. Secondly, if the irradiance is absorbed by other material than the hair it is not used for treatment. Therefore, in the embodiments of Fig. 1C-1E, the opposed LEDs are positioned "out of phase": a LED in one leg of the hair straightener will not face another LED in the other leg of the hair straightener. If reflective materials are used at the opposed blank spaces, photons can be recycled. As the reflected light preferably should not be guided back into the LED it came from, the reflective materials should reflect side-wards (herein, the notion side-wards covers all directions except for reflecting light back into the LED or LEDs from which the light originated).

Advantageously, in each leg, the zone between the parts of the heat bridge 22 that touch the hairs is provided with a transparent cover that is heated by the heat bridge 22, either from the side or because the cover also covers the heat bridge 22. In this way, the area in which heat can be applied to the hairs is increased. Preferably, the transparent cover is not fully transparent, in that it absorbs wavelengths > 1000 nm that are not useful for hair styling. This way wavelengths that are not effective to treat hairs are used to heat the treatment area, which heat is again useful for hair styling.

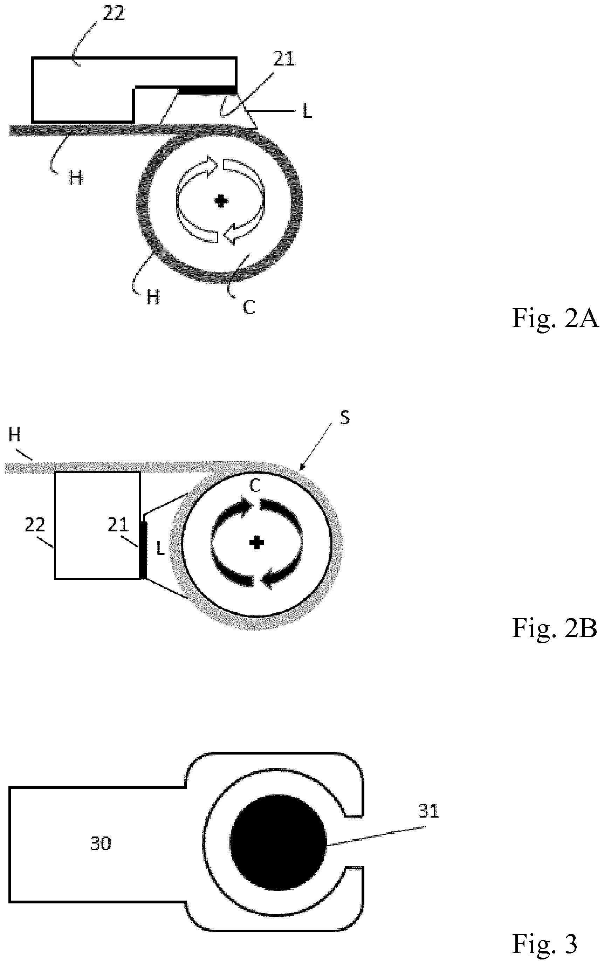

A second embodiment of the invention, shown in Fig. 2A, illustrates an auto-curler (e.g. of the type as shown in WO2016042476 or WO2017029382) in which according to the present invention, hair H is first guided along a heat bridge 22, before it is exposed to light L from a LED unit 21 and wrapped around a cylinder C. The heat bridge 22 ensures that the heat generated by the LED unit 21 is used to preheat the hair H before the hair is exposed to the light L from the LED unit 21. As the light L is only applied to the hair inside the auto-curler, this device is inherently safe as there is no risk of light reaching the eyes.

In an alternative embodiment as shown in Fig. 2B, the cylinder C is heated to a temperature somewhat less than 100 °C, preferably to a temperature between 70 °C and 90 °C. In this embodiment, the LED unit 21 is positioned such that hair H is first exposed to heat from the heat bridge 22 and the cylinder C. As the hair H is pre-heated by the heat bridge 22 and by the cylinder C, less optical energy from the LED unit 21 is needed to heat the heat to a temperature sufficiently hot for hair styling. A further improvement provides a sensor S, preferably an infrared sensor, to measure the hair temperature as results from pre-heating by the heat bridge 22 and the cylinder C, and a feedforward control device (not shown) controls the optical radiation source (LED unit 21) in dependence on a signal from the sensor S, to feed-forward control the LED unit 21. In the embodiment of Fig. 2B, part of the heat of the heat source 22, C originates from the LED unit 21.

Another embodiment of the invention, shown in Fig. 3, features a handheld hair curling device 30 (e.g. an auto-curler) comprising a light exposure unit 31 (e.g. rod-shaped) with light-emitting diodes (LEDs). The light exposure unit 31 is covered by a transparent material around which hair to be treated is wrapped. The inside of the hair curling device 30 around the light exposure unit 31 may be reflective to ensure that all emitted photons are optimally used for hair styling.

Option 1: In the transparent material a filter is included which is ideally completely transparent to the LED light emitted but absorbs the heat reflected or conducted via the hairs. The filter only works from the outside in. Not vice versa.

Option 2: In the transparent material a filter is included which is transparent for wavelengths smaller than 1000 nm (in particular between 400 nm and 1000 nm, i.e. the wavelengths useful for hair styling) and absorbs wavelengths > 1000 nm that are not useful for hair styling. This way wavelengths that are not effective to treat hairs are used to heat the treatment area, which heat is again useful for hair styling. In options 1 and 2, the absorbed heat heats the transparent material so that it forms a heat source for heating hair by conduction via direct contact between a hair contact surface of the heat source and the hair.

Option 3: In the transparent material a filter is included which is transparent to the LED light emitted while it is thermally connected to the heat sink of the LED. This way the transparent material is heated to temperatures around 60 to 100 °C so that it forms a heat source for heating hair by conduction via direct contact between a hair contact surface of the heat source and the hair.

Option 4: Around the surface of the light exposure unit 31, strips of LEDs alternate with strips of a heat sink of the LEDs that thereby form a heat source for heating hair by conduction via direct contact between a hair contact surface of the heat source and the hair. The strips are preferably oriented in the longitudinal direction of the rod-shaped light exposure unit 31.

One or more of the above options 1-4 may be combined.

In the embodiment of Fig. 4, a transparent cover 23 (e.g. made of polycarbonate or glass) covering the light engine(s) 21 is not fully transparent (80% transparent or higher) to the light. This way the cover 23 is heated which will (pre-)heat the hair via conduction. In another embodiment, the transparent cover 23 covering the light engine is only transparent to wavelengths between 400 and 1000 nm, i.e. the wavelengths that are useful for hair styling. Other wavelengths are absorbed in the cover 23 which will heat the cover plate. This heat is used to (pre-)heat the hairs via conduction, and so again in a way that is useful for hair styling. An extra advantage of using not fully transparent covers is that thereby, any moist escaping from the hairs when they are treated will not condensate on the covers which would hamper the flow of light energy to the hairs. As shown in Fig. 4, the cover 23 may cover the heat bridge 22 so that the cover 23 is heated also by the heat bridge 22. In that case, the heat source for heating hair by conduction via direct contact between a hair contact surface of the heat source and the hair comprises both the heat bridge 22 and the cover 23, with the cover forming the contact surface of the heat source.

In an embodiment, the system uses pulsed LEDs to style hair. The output wavelength is preferably in the range between 400 and 900 nm, and more preferably in the range between 450 and 550 nm. The pulse width is preferably shorter than or equal to 200 ms, and more preferably shorter than or equal to 100 ms. To prevent the hair from being damaged, the output energy fluence on the hair surface is preferably in the range between 1 J/cm² and 10 J/cm², more preferably between 3 J/cm² and 7 J/cm², and most preferably between 4 and 6 J/cm². The LED heat is guided towards the treatment area via a "heat bridge", and a light filter (absorber) may be included in the treatment area.

In sum, embodiments of the present invention feature technology to heat and therefore style hairs by both absorption and conduction. Primarily hairs are heated by absorption (optical energy). However, during light generation heat will be generated as well. The latter does not have to be wasted in the process: this heat is reused into the system to lower the optical system requirements or at least compensate for any efficiency decrease during heating of the LEDs.

As set out in more detail in a co-pending application EP17190266.1 entitled to the same priority date as the present application (attorney's ref.: 2017PF02406), incorporated by reference herein, embodiments of the present invention are related to a hair styling device comprising a light engine to deliver optical energy to hair, in which the hair styling device is arranged to allow moist escaping from the hair in response to optical energy being applied to the hair, to escape from the hair styling device. Preferably, the light engine is the sole energy source for hair styling. A ventilator may move the moist away from the light engine. A processor may control the light engine, in which case the ventilator may also serve to cool the processor and/or the light engine. The hair styling device may comprise clamping members arranged for allowing hair to be guided between and styled by the clamping members, at least one of the clamping members being provided with the light engine. At least one of the clamping members may be provided with openings for allowing moist to escape, or with openings for allowing air to enter so as to convey the moist out of the hair styling device. The clamping members may have non-conforming shapes to allow the moist to escape from the hair styling device. A hair treatment area comprising the light engine may have a gap through which the hair can be guided, the gap being sufficiently wide to allow the moist to escape. A width of the gap may be between 0.3 and 5 mm, and preferably between 1 and 2 mm.

As set out in more detail in a co-pending application EP17190268.7 entitled to the same priority date as the present application (attorney's ref.: 2017PF02407), embodiments of the present invention are related to a hair styling device that comprises an optical radiation source for radiating hair, a sensor unit for measuring effects from radiating hair, and a feedforward control device for controlling the optical radiation source in dependence on a signal from the sensor unit. The optical radiation source may produce a first flash having a first energy density that may be lower than required for photo-thermal hair reshaping, the optical radiation source being controlled to produce a subsequent flash in dependence on a sensor signal obtained in response to the first flash, which subsequent flash may have at least the first energy density. The sensor unit may include a sensor arranged before the optical radiation source in a hair flow direction. The hair styling device may comprise, along a direction in which the hair is guided, a first sensor, a first LED unit being controlled in dependence on a signal from the first sensor, a second sensor, and a second LED unit being controlled in dependence on a signal from the second sensor. The direction in which hair is guided through the hair styling device may determine which part of the optical radiation source will act as the first LED unit. The hair styling device may comprise a drive mechanism to move the hair along the optical radiation source at a speed controlled by the feedforward control device in dependence on the signal from the sensor unit.

As set out in more detail in a co-pending application EP17190269.5 entitled to the same priority date as the present application (attorney's ref.: 2017PF02408), embodiments of the present invention are related to a hair styling device having a two-dimensional array of elements to bring hair at a styling temperature, in which the elements produce optical radiation energy. The elements may include one or more LEDs, and preferably a plurality of LEDs, in which case the LEDS are driven in clusters that may be of mutually different shapes and sizes. The hair styling device may comprise sensors to obtain an areal light absorption measurement opposed to the two-dimensional array of elements, and a control unit for individually controlling the elements in dependence of the measurement. The hair styling device may radiate hair from two sides, both of which includes an areal light absorption measurement. The sensors may include LEDs that momentarily do not produce light.

It should be noted that the above-mentioned embodiments illustrate rather than limit the invention, and that those skilled in the art will be able to design many alternative embodiments without departing from the scope of the appended claims. The invention is limited by the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. A control unit for controlling the optical radiation source may be implemented by means of hardware comprising several distinct elements, and/or by means of a suitably programmed processor. In the device claim enumerating several means, several of these means may be embodied by one and the same item of hardware. The mere fact that certain measures are recited in mutually different dependent claims that do not refer to one another does not indicate that a combination of these measures cannot be used to advantage.

## Claims

1. A hair styling device (20) comprising:
a heat source (22, 23, C) for heating hair by conduction via direct contact between a hair contact surface of the heat source (22, 23, C) and the hair, and
an optical radiation source (21) for - in combination with heat from the heat source (22, 23, C) - heating the hair to a temperature sufficiently high for hair styling by absorption of optical radiation by the hair,
**characterized in that** the heat source (22, 23, C) obtains heat from energy provided by the optical radiation source (21).

2. A hair styling device (20) as claimed in claim 1, wherein the heat source (22, 23, C) includes a heat bridge (22) coupled to a heat sink of the optical radiation source (21), the heat bridge (22) having the hair contact surface for heating the hair.

3. A hair styling device (20) as claimed in claim 2, wherein the heat bridge (22) is arranged for heating the hair (H) before the hair (H) is exposed to light from the optical radiation source (21).

4. A hair styling device (20) as claimed in any of the preceding claims, wherein the optical radiation source (21) is arranged for radiating hairs using one radiation flash having a duration of at least 0.1 s.

5. A hair styling device (20) as claimed in claim 1, 2 or 3, wherein the optical radiation source (21) is arranged for radiating hairs using at least two radiation flashes, an interval between subsequent flashes being smaller than 5 s, preferably smaller than 1 s, and more preferably smaller than 0.3 s.

6. A hair styling device (20) as claimed in any of the preceding claims, wherein the optical radiation source (21, 31) is covered by a cover (23) that is not fully transparent, whereby optical radiation energy is transformed into thermal energy, the heat source (22, 23, C) including the cover (23), the cover (23) having the hair contact surface for heating the hair.

7. A hair styling device as claimed in claim 6, wherein the cover (23) is largely transparent for wavelengths effective for hair styling, while the cover (23) is largely not transparent for wavelengths less effective for hair styling.

8. A hair styling device as claimed in any of the preceding claims 1-5, wherein the optical radiation source (21) is covered by a cover (23) that is heated by the heat source (22).

9. A hair styling device as claimed in any of the preceding claims, and having first and second parts between which hair is positioned, each part having respective optical radiation sources (21), wherein optical radiation sources in the first part do not face optical radiation sources in the second part.

10. A hair styling device as claimed in claim 9, wherein in each part, reflective materials are positioned between adjacent optical radiation sources for reflecting light side-wards.

11. A hair styling device as claimed in any of the preceding claims, wherein an output energy fluence on a hair surface is in a range between 1 J/cm² and 10 J/cm², preferably between 3 J/cm² and 7 J/cm², and more preferably between 4 and 6 J/cm².

12. A hair styling device as claimed in any of the preceding claims, the heat source (22, 23, C) comprising a heater (C) for pre-heating the hair (H) up till a first temperature that does not exceed 100 °C, and preferably lies between 70 °C and 90 °C, before the hair (H) is exposed to light from the optical radiation source (21).

## Patentansprüche

1. Haarstylingvorrichtung (20), umfassend:
eine Wärmequelle (22, 23, C) zum Erhitzen von Haaren durch Wärmeleitung über den direkten Kontakt zwischen einer Haarkontaktfläche der Wärmequelle (22, 23, C) und den Haaren und
eine optische Strahlungsquelle (21) zum Erhitzen - in Kombination mit Wärme von der Wärmequelle (22, 23, C) - der Haare auf eine Temperatur, die für das Haarstyling durch Absorption optischer Strahlung durch die Haare ausreichend hoch ist,
**dadurch gekennzeichnet, dass** die Wärmequelle (22, 23, C) Wärme aus Energie erhält, die von der optischen Strahlungsquelle (21) bereitgestellt wird.

2. Haarstylingvorrichtung (20) nach Anspruch 1, wobei die Wärmequelle (22, 23, C) eine Wärmebrücke (22) umfasst, die mit einem Kühlkörper der optischen Strahlungsquelle (21) gekoppelt ist, wobei der Wärmebrücke (22) die Haarkontaktfläche zum Erhitzen der Haare aufweist.

3. Haarstylingvorrichtung (20) nach Anspruch 2, wobei die Wärmebrücke (22) zum Erhitzen der Haare (H) angeordnet ist, bevor die Haare (H) dem Licht der optischen Strahlungsquelle (21) ausgesetzt werden.

4. Haarstylingvorrichtung (20) nach einem der vorhergehenden Ansprüche, wobei die optische Strahlungsquelle (21) zum Strahlen von Haaren unter Verwendung eines Strahlungsblitzes mit einer Dauer von mindestens 0,1 s angeordnet ist.

5. Haarstylingvorrichtung (20) nach Anspruch 1, 2 oder 3, wobei die optische Strahlungsquelle (21) zum Strahlen von Haaren unter Verwendung von mindestens zwei Strahlungsblitzen angeordnet ist, wobei ein Intervall zwischen nachfolgenden Blitzen kleiner als 5 s, vorzugsweise kleiner als 1 s und bevorzugter kleiner als 0,3 s ist.

6. Haarstylingvorrichtung (20) nach einem der vorhergehenden Ansprüche, wobei die optische Strahlungsquelle (21, 31) von einer Abdeckung (23) bedeckt ist, die nicht vollständig transparent ist; wobei optische Strahlungsenergie in Wärmeenergie umgewandelt wird, wobei die Wärmequelle (22, 23, C) die Abdeckung (23) enthält, wobei die Abdeckung (23) die Haarkontaktfläche zum Erhitzen der Haare aufweist.

7. Haarstylingvorrichtung nach Anspruch 6, wobei die Abdeckung (23) für Wellenlängen, die für das Haarstyling wirksam sind, weitgehend transparent ist, während die Abdeckung (23) für Wellenlängen, die für das Haarstyling weniger wirksam sind, weitgehend nicht transparent ist.

8. Haarstylingvorrichtung nach einem der vorhergehenden Ansprüche 1 bis 5, wobei die optische Strahlungsquelle (21) von einer Abdeckung (23) bedeckt ist, die von der Wärmequelle (22) erwärmt wird.

9. Haarstylingvorrichtung nach einem der vorhergehenden Ansprüche und mit ersten und zweiten Teilen, zwischen denen Haare positioniert sind, wobei jeder Teil entsprechende optische Strahlungsquellen (21) aufweist, wobei optische Strahlungsquellen im ersten Teil keinen optischen Strahlungsquellen im zweiten Teil gegenüberstehen.

10. Haarstylingvorrichtung nach Anspruch 9, wobei in jedem Teil reflektierende Materialien zwischen benachbarten optischen Strahlungsquellen positioniert sind, um Licht seitlich zu reflektieren.

11. Haarstylingvorrichtung nach einem der vorhergehenden Ansprüche, wobei eine Ausgangsenergiefluenz auf einer Haaroberfläche in einem Bereich zwischen 1 J/cm² und 10 J/cm² liegt, vorzugsweise zwischen 3 J/cm² und 7 J/cm² und bevorzugter zwischen 4 und 6 J/cm².

12. Haarstylingvorrichtung nach einem der vorhergehenden Ansprüche, wobei die Wärmequelle (22, 23, C) eine Heizung (C) zum Vorheizen der Haare (H) bis zu einer ersten Temperatur umfasst, die 100°C nicht überschreitet und vorzugsweise zwischen 70°C und 90°C liegt, bevor die Haare (H) dem Licht der optischen Strahlungsquelle (21) ausgesetzt werden.

## Revendications

1. Dispositif de coiffure (20) comprenant:
une source de chaleur (22, 23, C) pour chauffer les cheveux par conduction via un contact direct entre une surface de contact avec les cheveux de la source de chaleur (22, 23, C) et les cheveux, et
une source de rayonnement optique (21) pour - en combinaison avec la chaleur de la source de chaleur (22, 23, C) - chauffer les cheveux à une température suffisamment élevée pour la coiffure par absorption du rayonnement optique par les cheveux,
**caractérisé en ce que** la source de chaleur (22, 23, C) obtient de la chaleur à partir de l'énergie fournie par la source de rayonnement optique (21).

2. Dispositif de coiffure (20) selon la revendication 1, dans lequel la source de chaleur (22, 23, C) comprend un pont thermique (22) couplé à un dissipateur thermique de la source de rayonnement optique (21), le pont thermique (22) ayant la surface de contact avec les cheveux pour chauffer les cheveux.

3. Dispositif de coiffure (20) selon la revendication 2, dans lequel le pont thermique (22) est agencé pour chauffer les cheveux (H) avant que les cheveux (H) soient exposés à la lumière de la source de rayonnement optique (21).

4. Dispositif de coiffure (20) selon l'une quelconque des revendications précédentes, dans lequel la source de rayonnement optique (21) est agencée pour rayonner les cheveux en utilisant un flash de rayonnement d'une durée d'au moins 0,1 s.

5. Dispositif de coiffure (20) selon la revendication 1, 2 ou 3, dans lequel la source de rayonnement optique (21) est agencée pour rayonner les cheveux en utilisant au moins deux flashes de rayonnement, un intervalle entre des flashes suivants étant inférieur à 5 s, de préférence inférieur à 1 s, et plus préférablement inférieur à 0,3 s.

6. Dispositif de coiffure (20) selon l'une quelconque des revendications précédentes, dans lequel la source de rayonnement optique (21, 31) est recouverte par un couvercle (23) qui n'est pas totalement transparent, grâce à quoi l'énergie de rayonnement optique est transformée en énergie thermique, la source de chaleur (22, 23, C) comprenant le couvercle (23), le couvercle (23) ayant la surface de contact avec les cheveux pour chauffer les cheveux.

7. Dispositif de coiffure selon la revendication 6, dans lequel le couvercle (23) est largement transparent pour les longueurs d'onde efficaces pour la coiffure, tandis que le couvercle (23) est largement non transparent pour les longueurs d'onde moins efficaces pour la coiffure.

8. Dispositif de coiffure selon l'une quelconque des revendications 1 à 5 précédentes, dans lequel la source de rayonnement optique (21) est recouverte par un couvercle (23) qui est chauffé par la source de chaleur (22) .

9. Dispositif de coiffure selon l'une quelconque des revendications précédentes, et ayant des première et seconde parties entre lesquelles les cheveux sont positionnés, chaque partie ayant des sources de rayonnement optique respectives (21), où les sources de rayonnement optique dans la première partie ne font pas face à les sources de rayonnement optique dans la seconde partie.

10. Dispositif de coiffure selon la revendication 9, dans lequel dans chaque partie, des matériaux réfléchissants sont positionnés entre des sources de rayonnement optique adjacentes pour réfléchir de la lumière latéralement.

11. Dispositif de coiffure selon l'une quelconque des revendications précédentes, dans lequel une fluence d'énergie de sortie sur une surface capillaire est dans une plage entre 1 J/cm² et 10 J/cm², de préférence entre 3 J/cm² et 7 J/cm², et plus préférentiellement entre 4 et 6 J/cm².

12. Dispositif de coiffure selon l'une quelconque des revendications précédentes, la source de chaleur (22, 23, C) comprenant un élément chauffant (C) pour préchauffer les cheveux (H) jusqu'à une première température qui ne dépasse pas 100°C, et se situe de préférence entre 70°C et 90°C, avant que les cheveux (H) soient exposés à la lumière provenant de la source de rayonnement optique (21).
